(19) Europäisches Patentamt
European Patent Office
Office européen des brevets

(11) Publication number: **0 076 085**
**A1**

(12) **EUROPEAN PATENT APPLICATION**

(21) Application number: 82304973.9

(22) Date of filing: 21.09.82

(51) Int. Cl.³: C 07 D 241/12
C 07 D 241/42

(30) Priority: 30.09.81 US 307262

(43) Date of publication of application:
06.04.83 Bulletin 83/14

(84) Designated Contracting States:
DE GB NL

(71) Applicant: PHILIP MORRIS INCORPORATED
100 Park Avenue
New York, New York 10017(US)

(72) Inventor: Williams, David Lee
10112 Cutter Drive
Richmond Virginia 23235(US)

(72) Inventor: Houminer, Yoram
10107 Windsong Terrace
Richmond Virginia 23233(US)

(72) Inventor: Southwick, Everett West
634 Elgin Terrace
Richmond Virginia 23225(US)

(74) Representative: Bass, John Henton et al,
REDDIE & GROSE 16 Theobalds Road
London WC1X 8PL(GB)

(54) **Production of monoacylpyrazines.**

(57) This invention provides a process for preparing monoacylpyrazines which involves coreacting an aldehyde (R-CHO) and a pyrazine compound corresponding to the formula:

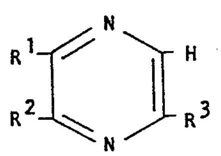

where $R^1$, $R^2$ and $R^3$ are substituents selected from hydrogen and alkyl groups, and $R^1$ and $R^2$ when taken together with connecting elements may form an alicyclic or aromatic structure, and R in the aldehyde compound is selected from aliphatic, alicyclic and aromatic groups under free radical conditions in a heterogeneous reaction medium consisting of an organic phase and an aqueous phase. The compounds prepared have the formula:

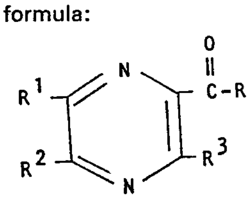

where R, $R^1$, $R^2$ and $R^3$ are as previously defined, which is then recovered.

EP 0 076 085 A1

# PRODUCTION OF MONOACYLPYRAZINES

## BACKGROUND OF THE INVENTION

Unlike alkylpyrazines which are ubiquitous in nature and heat-treated foodstuffs, acylpyrazines are more limited in their occurrence. For example, 2-acetyl-5-methylpyrazine and 2-acetyl-5-ethylpyrazine are reported as constituents of cocoa in Tobacco International, page 18ff (March 1979), and 1-(2-pyrazinyl)-1-butanone is tentatively identified as a water-soluble component of cigarette smoke in J. Agric. Food Chem., 25(2), 310 (1977).

Several acetylpyrazines are included in the F.E.M.A. listing of food additives as being useful for imparting a popcorn-nutty flavor to a foodstuff. The incorporation of acetylpyrazine, 2-acetyl-5-methylpyrazine or 2-acetyl-6-methylpyrazine as a popcorn-like flavorant in foodstuffs and tobacco is described in U.S. 3,402,051. Interest in substituted pyrazines such as acylpyrazines as flavorants in foodstuffs and tobacco has stimulated the investigation of synthesis methods for the preparation of this useful class of heterocyclic compounds.

U.S. 3,711,482 describes a process for preparing 2-acetyl-3-alkylpyrazine which involves reacting 2-ethyl-3-alkylpyrazine with N-bromosuccinimide to provide 2-(1-bromoethyl)-3-alkylpyrazine,and then treating the bromoethylalkylpyrazine with an alkali metal and 2-nitropropane to yield 2-acetyl-3-alkylpyrazine.

U.S. 3,767,428 describes a process for preparing 2-acetyl-3-ethylpyrazine which involves reacting 2,3-diethylpyrazine with peracetic acid in an acetic acid medium at 70-80°C to provide 2-ethyl-3-(1-acetoxyethyl)pyrazine. Treatment of this intermediate product with potassium hydroxide yields 2-ethyl-3-(1-hydroxyethyl)pyrazine, which is subsequently oxidized with dimethylsulfoxide and acetic anhydride to give the desired 2-acetyl-3-ethylpyrazine.

U.S. 3,890,320 describes a process for preparing 2-acyl-3-alkylpyrazines which involves treating 2-alkyl-3-alkylpyrazine with an alkali metal dichromate in an acidic medium, and recovering 2-acyl-3-alkylpyrazine product.

U.S. 3,914,227 describes a process which involves reacting methylpyrazine anion with 2-ethylcyclohexylbromide to provide a 2-(2-ethylcyclohexyl)methylpyrazine intermediate product, and then admixing the intermediate product with N-bromosuccinimide, carbon tetrachloride and benzoylperoxide to produce cyclohexyl pyrazine ketone.

In J. Chem. Soc., Perkin II, 2035 (1972) there is reported the acylation of protonated pyrazine derivatives. In a general procedure, a heteroaromatic compound (e.g., pyrazine) is acylated by reacting the compound with alkanal in the presence of t-butyl hydroperoxide and iron(II) sulfate in a homogeneous aqueous medium of acetic acid and sulfuric acid. Under these conditions, the heteroaromatic compound is polyacylated to yield the following type of pyrazine derivative:

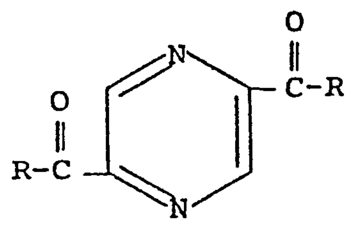

The diacyl derivative is readily obtained with an excess of acylating agent, whereas the monoacyl derivatives prevail only at very low conversions.

Hence, the methods developed for the production of monoacylpyrazine derivatives involve either cumbersome oxidative conversion of alkylpyrazines, or acylation of pyrazines under low conversion conditions.

Accordingly, it is an object of this invention to provide a general and efficient process for selective acylation of pyrazines to monoacyl derivatives.

It is another object of this invention to provide novel monoacyl pyrazine compounds which have aroma and flavor characteristics which range from a low butterscotch note to sweet and cheesy.

Other objects and advantages of the present invention shall become apparent from the accompanying description and examples.

DESCRIPTION OF THE INVENTION

One or more objects of the present invention are accomplished by the provision of a process for preparing monoacylpyrazines which comprises the steps of (1) providing a heterogeneous reaction medium consisting of a water-immiscible organic phase and an acidic aqueous phase, wherein the organic phase comprises a mixture of an aldehyde compound (RCHO) and a pyrazine compound corresponding to the formula:

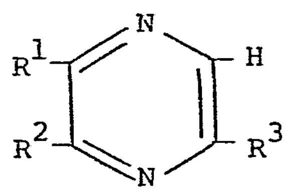

where $R^1$, $R^2$ and $R^3$ are substituents selected from hydrogen and alkyl groups, and $R^1$ and $R^2$ when taken together with connecting elements may form an alicyclic or aromatic structure, and R in the aldehyde compound is a substituent selected from aliphatic, alicyclic and aromatic groups; (2) maintaining efficient contact between the organic and aqueous phases for a period of time sufficient to achieve acylation of the pyrazine compound in the presence of a free radical generating agent; and (3) recovering a monoacylpyrazine product corresponding to the formula:

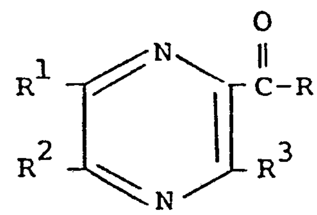

where R, $R^1$, $R^2$ and $R^3$ are substituents as previously defined.

The aldehyde (R-CHO) reactant can be any compound which does not contain any substituents which inhibit or prevent the free radical interaction of the aldehyde functionality with the pyrazine nucleus. It is preferred that the aldehyde reactant is at least partially soluble in the aqueous phase of the acylation system, in order to increase the rate and efficiency of the acylation reaction.

Illustrative of radicals corresponding to R in the above formula are organic substituents containing between about 1-12 carbon atoms such as methyl, ethyl, propyl, isopropyl, butyl, butenyl, isobutyl, pentyl, octyl, decyl, chloroethyl, cyclopentyl, cyclohexyl, cyclohexenyl, phenyl, chlorophenyl, methoxyphenyl, tolyl, xylyl, naphthyl, furyl, thienyl, and the like.

Illustrative of suitable pyrazine compounds corresponding to the formula above are those in which $R^1$, $R^2$ and $R^3$ are substituents selected from hydrogen and alkyl groups containing between about 1-8 carbon atoms such as methyl, ethyl, propyl, butyl, isobutyl, tertiary-butyl, pentyl, and the like.

When taken together with connecting elements, $R^1$ and $R^2$ can form an alicyclic or aromatic structure. Illustrative of this type of pyrazine derivative are quinoxaline and 5,6,7,8-tetrahydroquinoxaline:

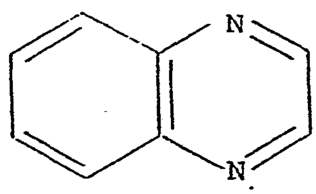
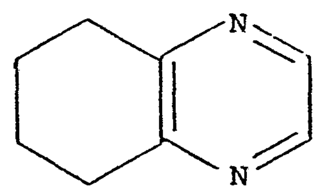

The pyrazine and aldehyde reactants can be employed over a wide range of molar ratios. It has been found convenient and advantageous to employ a molar ratio between about 0.5-10:1 of aldehyde to pyrazine in the acylation system.

It is an essential aspect of the invention process that the acylation system is heterogeneous, i.e., the acylation medium is composed of a water-immiscible organic phase in intimate contact with an acidic aqueous phase. Efficient contact of the two phases is achieved by suitable means of stirring or agitation.

The organic phase usually will comprise a mixture of the pyrazine and aldehyde reactants. If the water-solubility of the pyrazine and aldehyde reactants are at a sufficiently high level to prevent the maintenance of separate organic and aqueous phases, then suitable modification of the acylation system is required to accomplish the separation of phases. Cooling of the acylation system is one means of

providing the desired phase separation, particularly in combination with other means such as salting of the aqueous phase. A preferred alternative is to include a water-immiscible solvent in the system, such as hexane, benzene or toluene.

The relative volumes of the respective immiscible phases in the acylation system are not critical, and typically the two phases will be approximately equal in volume.

The acidity of the aqueous phase is provided by the addition of a suitable acid reagent such as sulfuric acid, hydrochloric acid, phosphoric acid, and the like. The pH of the aqueous phase is below about 6, and preferably is in the range between about 1-5.

The acylation reaction between the pyrazine and aldehyde reactants is catalyzed by the inclusion of a free radical generating agent, in a quantity between about 1-50 weight percent, based on the weight of aldehyde reactant.

Illustrative of suitable free radical initiators are hydrogen peroxide; alkali metal or ammonium persulfates, perborates, peracetates and percarbonates; organic peroxides and hydroperoxides such as benzoyl peroxide, t-butylhydroperoxide and diisopropylperoxydicarbonate; and the like. The initiator may be associated with activating means (e.g., a redox system) which involves the use of compounds such as sulfites and thiosulfites, and redox reaction promoters such as transition metal ions (e.g., $Fe^{++}$).

In a typical procedure the monoacylation reaction is conducted for a time period between about 0.2-2 hours with efficient stirring of the heterogeneous phases.

Atmospheric pressure is usually employed and the temperature of the monoacylation is maintained in the range between about 0°-60°C.

After the completion of the reaction, the monoacylpyrazine product is recovered by conventional procedures. If a water-immiscible solvent is employed during the monoacylation reaction, the solvent-containing organic phase is separated and then fractionated by distillation to recover monoacylpyrazine product. Preferably, the organic phase is washed with aqueous alkaline solution before the distillation.

The separate aqueous phase is extracted with a solvent, and after the solvent extract phase is washed with aqueous alkaline solution; the solvent extract phase is distilled to yield an additional quantity of monoacylpyrazine product.

If no organic solvent is employed as a water-immiscible diluent during the monoacylation reaction period, then recovery of monoacylpyrazine product can be accomplished, for example, by extracting the resultant heterogeneous phase reaction product mixture with a water-immiscible solvent, followed by washing the solvent extract phase with aqueous alkaline solution, and distilling the solvent extract under vacuum to isolate the desired monoacylpyrazine product. The

said product can be further subjected to purification techniques, such as distillation, preparative gas liquid chromatography, thick layer chromatography or crystallization as appropriate for a particular monoacylpyrazine product.

The following examples are further illustrative of the present invention. The reactants and other specific ingredients are presented as being typical, and various modifications can be devised in view of the foregoing disclosure within the scope of the invention.

## EXAMPLE I

### Preparation Of 1-Pyrazinyl-3-Methyl-1-Butanone

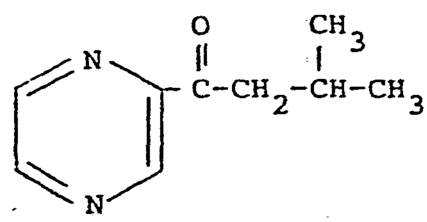

To a stirring heterogeneous mixture of isovaleraldehyde (5.16 grams, 60 mmoles) and pyrazine (801 milligrams, 10 mmoles) in 5 milliliters of 3.4M sulfuric acid, at 3°-5°C, are added concurrently 70% t-butylhydroperoxide (5.4 grams, 42 mmoles) and a solution of ferrous sulfate (16.7 grams, 60 mmoles) in 40 milliliters of water over a 15 minute period. The resulting heterogeneous mixture is stirred an additional 1 hour, during which time the temperature is raised to 15°C. Solid sodium sulfite is then added until test with starch-iodide paper is negative.

The aqueous mixture is extracted with methylene chloride (3 x 100 milliliters), and the extracts are combined and washed with water. Optionally, the combined extract phase is washed with aqueous alkaline solution to remove acidic components. After drying ($MgSO_4$), the solvent is removed under reduced pressure to yield 2.5 grams of crude reaction mixture.

Analysis by gas chromatography indicates two major components in a 3:1 ratio, comprising the desired 1-pyrazinyl-3-methyl-1-butanone and isobutylpyrazine (identified by MS). The amount of diacyl byproduct is negligible. Preparative thick layer chromatography (2000u silica gel GF, developed with methylene chloride) provides a 12% yield of 1-pyrazinyl-3-methyl-1-butanone.

An analytically pure sample for odor and flavor evaluation is obtained by preparative GLC (1/4" x 15' Carbowax ® 20M-TPA). The structure is verified by IR, NMR and MS spectroscopy.

| | | |
|---|---|---|
| Anal. Calc. for $C_9H_{12}N_2O$: | | C, 65.83; H, 7.37; N, 17.06 |
| | Found: | C, 65.61; H, 7.22; N, 16.82 |

EXAMPLE II

Preparation Of 1-Pyrazinyl-1-Propanone

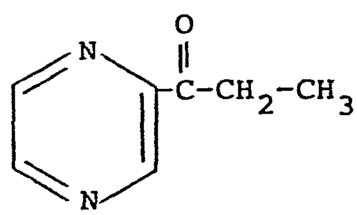

The reaction of pyrazine and freshly distilled propionaldehyde is conducted in the manner described in Example I on the same molar scale. The crude reaction mixture is flash-distilled and the volatile fraction sublimed (1mm Hg at 55°C) to give a 29% yield of 1-pyrazinyl-1-propanone (mp 46-47°C).

An analytically pure sample for odor and flavor evaluation is obtained by preparative GLC (1/4" x 15' Carbowax® 20M-TPA). The structure is verified by spectroscopy.

| | | |
|---|---|---|
| Anal. Calc. for $C_7H_8N_2O$: | | C, 61.75; H, 5.92; N, 20.58 |
| | Found: | C, 61.55; H, 5.81; N, 20.41 |

Also isolated by gas chromatography (GC) from the reaction mixture is 2.1% of 2,5-diacyl product, the structure of which is verified by NMR.

EXAMPLE III

Preparation Of 1-Pyrazinyl-2,2-Dimethyl-1-Propanone

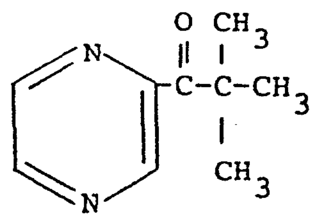

The reaction of pyrazine and trimethylacetaldehyde is conducted in the manner described in Example I on the same molar scale. Preparative thick layer chromatography (2000µ silica gel GF, developed with methylene chloride) of the crude reaction product provides an 11% yield of 1-pyrazinyl-2,2-dimethyl-1-propanone.

An analytically pure sample for odor and flavor evaluation is obtained by preparative GLC (1/4" x 15' Carbowax® 20M-TPA). The structure is verified by spectroscopy.

| | |
|---|---|
| Anal. calc. for $C_9H_{12}N_2O$: | C, 65.83; H, 7.37; N, 17.06 |
| Found: | C, 65.80; H, 7.50; N, 16.87 |

Also detected by GC is a trace amount of a product tentatively identified as a diacyl derivative.

EXAMPLE IV

Preparation Of 1-Pyrazinyl-2-Methyl-1-Propanone

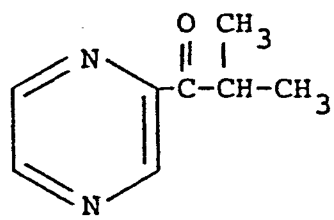

The reaction of pyrazine and isobutyraldehyde is conducted in the manner described in Example I on the same molar scale. Preparative thick layer chromatography (2000µ silica gel GF, developed with methylene chloride) of the crude reaction product provides a 24.3% yield of 1-pyrazinyl-2-methyl-1-propanone.

An analytically pure sample for odor and flavor evaluation is obtained by preparative GLC (1/4" x 15' Carbowax® 20M-TPA). The structure is verified by spectroscopy.

Anal. calc. for $C_8H_{10}N_2O$: C, 63.98; H, 6.72; N, 18.65

Found: C, 64.07; H, 6.61; N, 18,42

Also detected by GC is a trace amount of a product tentatively identified as a diacyl derivative.

EXAMPLE V

Preparation Of 1-Pyrazinyl-1-Butanone

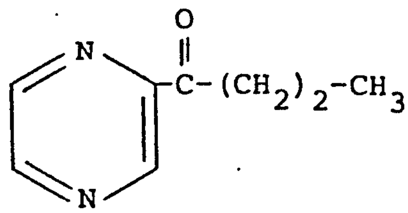

The reaction of pyrazine and butyraldehyde is conducted in the manner described in Example I on the same molar scale. Preparative thick layer chromatography (2000μ silica gel GF, developed in 5% acetone/hexane) of the crude reaction product provides a 25% yield of 1-pyrazinyl-1-butanone.

An analytically pure sample for odor and flavor evaluation is obtained by preparative GLC (1/4" x 15' Carbowax® 20M-TPA). The structure is verified by spectroscopy.

Anal. calc. for $C_8H_{10}N_2O$: C, 63.98; H, 6.72; N, 18.65

Found: C, 63.69; H, 6.86; N, 18.87

EXAMPLE VI

Preparation Of 1-(2,3-Dimethyl-5-Pyrazinyl)-1-Propanone

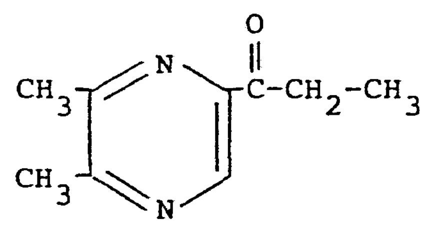

The reaction of 2,3-dimethylpyrazine and freshly distilled propionaldehyde is carried out as described in Example I on the same molar scale. Preparative thick layer chromatography (2000µ silica gel GF, developed in 5% acetone/hexane) of the crude reaction product gives a 25% yield of 1-(2,3-dimethyl-5-pyrazinyl)-1-propanone.

An analytically pure sample for odor and flavor evaluation is obtained by preparative GLC (1/4" x 15' Carbowax® 20M-TPA). The structure is verified by spectroscopy.

| | |
|---|---|
| Anal. calc. for $C_9H_{12}N_2O$: | C, 65.83; H, 7.37; N, 17.06 |
| Found: | C, 65.75; H, 7.50; N, 17.20 |

Also detected by GC is a trace amount of a product tentatively identified as a diacyl derivative.

EXAMPLE VII

Preparation Of 1-(2,3,5-Trimethyl-6-pyrazinyl)-1-propanone

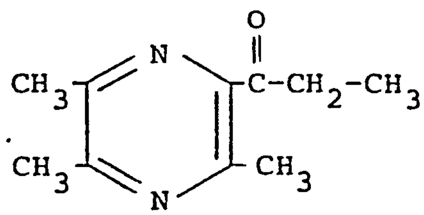

The reaction of trimethylpyrazine and freshly distilled propionaldehyde is carried out as described in Example I on the same molar scale. Preparative thick layer chromatography (2000µ silica gel GF, developed in 5% acetone/hexane) of the crude reaction product gives a 48% yield of 1-(2,3,5-trimethyl-6-pyrazinyl)-1-propanone (mp 65-67°C).

An analytically pure sample for odor and flavor evaluation is obtained by preparative GLC (1/4" x 15' Carbowax® 20M-TPA). The structure is verified by spectroscopy.

| | | |
|---|---|---|
| Anal. calc. for $C_{10}H_{14}N_2O$: | | C, 67.39; H, 7.92; N, 15.72 |
| | Found: | C, 67.24; H, 8.13; N, 15.52 |

## EXAMPLE VIII

Preparation Of 1-(3,5-Dimethyl-2-Pyrazinyl)-1-Propanone

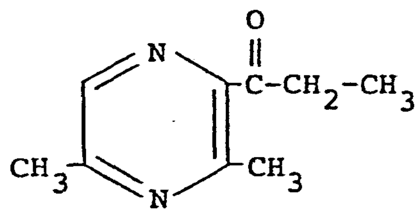

The reaction of 2,6-dimethylpyrazine and freshly distilled propionaldehyde is carried out as described in Example I on the same molar scale. Preparative thick layer chromatography (2000µ silica gel GF, developed in 5% acetone/hexane) of the crude reaction product gives a 39% yield of 1-(3,5-dimethyl-2-pyrazinyl)-1-propanone.

An analytically pure sample for odor and flavor evaluation is obtained by preparative GLC (1/4" x 15' Carbowax® 20M-TPA). The structure is verified by spectroscopy.

| | |
|---|---|
| Anal. calc. for $C_9H_{12}N_2O$: | C, 65.83; H, 7.37; N, 17.06 |
| Found: | C, 65.59; H, 7.50; N, 16.88 |

Also detected by GC is a trace amount of a product tentatively identified as a diacyl derivative.

EXAMPLE IX

Preparation Of 2-Acetyl-5,6,7,8-Tetrahydroquinoxaline

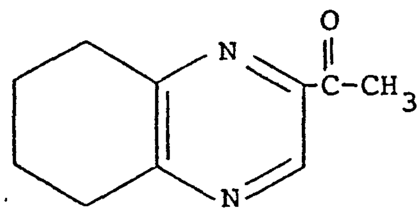

The reaction of 5,6,7,8-tetrahydroquinoxaline and freshly distilled acetaldehyde is carried out as described in Example I on one-half the molar scale. Preparative thick layer chromatography (2000µ silica gel GF, developed in 5% acetone/hexane) of the crude reaction product gives a 22% yield of 2-acetyl-5,6,7,8-tetrahydroquinoxaline.

An analytically pure sample for odor and flavor evaluation is obtained by preparative GLC (1/4" x 15' Carbowax® 20M-TPA). The structure is verified by spectroscopy.

Anal. calc. for $C_{10}H_{12}N_2O$: C, 68.16; H, 6.86; N, 15.90

Found: C, 68.01; H, 6.90; N, 15.74

EXAMPLE X

Preparation Of 1-[3-(2-Methyl-1-propyl)-2-pyrazinyl]-1-propanone, 1-[5-(2-Methyl-1-propyl)-2-pyrazinyl]-1-propanone, 1-[6-(2-Methyl-1-propyl)-2-pyrazinyl]-1-propanone.

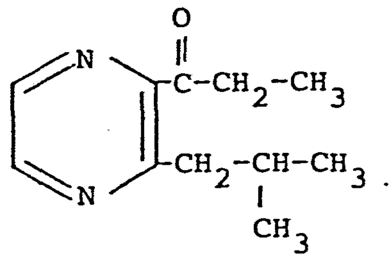

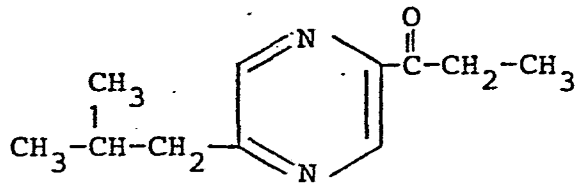

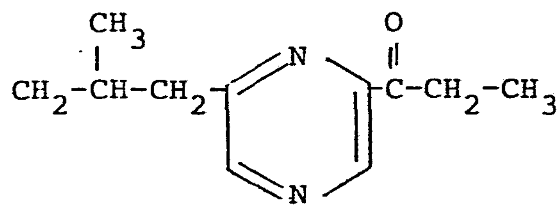

The reaction of isobutylpyrazine and freshly distilled propionaldehyde is carried out as described in Example I on the same molar scale. Preparative thick layer chromatography (2000μ silica gel GF, developed in 5% acetone/hexane) of the crude reaction product gives a 29% yield of a mixture of the three isomers.

Analytically pure samples of each isomer for odor and flavor evaluation are obtained by preparative GLC (1/4" x 15' Carbowax® 20M-TPA). The 2,3-; 2,5-; and 2,6-isomer ratio is found to be 3:3:4 respectively. The structures are verified by IR, NMR and MS spectroscopy.

Also isolated by GC from the reaction mixture are small amounts of diacyl derivatives, which are verified by MS and NMR.

EXAMPLE XI

Preparation Of 2-Pyrazinyl p-Methoxyphenyl Ketone

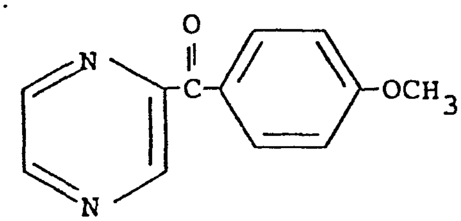

The reaction of pyrazine and p-methoxybenzaldehyde is carried out as described in Example I on the same molar scale. Successive preparative thick layer chromatography, (2000µ silica gel GF, developed with 15% acetone/hexane), of the crude reaction product gives a 1.5% yield of pyrazinyl p-methoxyphenyl ketone.

An analytically pure sample for odor and flavor evaluation is obtained by preparative GLC (1/4" x 6' 5% SE-30 on Chrom G-HP). The structure is verified by spectroscopy.

EXAMPLE XII

This Example illustrates the preparation of 2,5-dipropionylpyrazine in accordance with the procedure described in J. Chem. Soc., Perkins II, 2035(1972) by Coronna et al (as disclosed in the Background Of The Invention section of the present specification).

A homogeneous solution of propionaldehyde (2.9 grams, 50 mmoles), pyrazine (800 milligrams, 100 mmoles), glacial acetic acid (15 milliliters), conc. sulfuric acid (3 milliliters) and water (15 milliliters) is set stirring at 3°C. To this solution is added a solution of 5.56 grams of ferrous sulfate (20 mmole) in 10 milliliters of water followed by the rapid addition of 2.56 grams of 70% t-butylhydroperoxide (20 mmole).

After 5 minutes, a small amount of solid is noted on the surface of the reaction medium. The reaction is stirred for a total of 1 hour, during which time the reaction temperature is allowed to rise to 15°C. The solid product is isolated by filtration through a sintered glass funnel to give about 950 milligrams of 50% pure product. Purification by crystallization from hexane provided 150 milligrams of pure diacylproduct. The yield was calculated to be 25% based on the crude product.

| | | |
|---|---|---|
| Anal. calc. for $C_{10}H_{12}N_2O_2$: | | C, 62.48; H, 6.29; N, 14.58 |
| | Found: | C, 62.75; H, 6.33; N, 14.81 |

A negligible amount of the monoacyl product (<1%) was obtained by neutralizing the filtrate with 10% sodium hydroxide and extracting with methylene chloride.

EXAMPLE XIII

This Example illustrates the product selectivity advantage of conducting a pyrazine acylation reaction in a heterogeneous reaction medium as compared to a homogeneous system for the production of 1-pyrazinyl-1-propanone.

The reaction described in Example II is repeated employing a longer reaction time (total of 2 hours). An identical reaction is run with the exception that 40 milliliters of benzene are added prior to the addition of the ferrous sulfate and t-butylhydroperoxide.

After 2 hours the benzene layer is separated and the aqueous layer is extracted with 2 x 50 milliliters of methylene chloride. Each organic phase is dried ($MgSO_4$) and concentrated at reduced pressure. The benzene phase yields 0.65 gram of crude product, and the methylene chloride extract phase yields 0.1 gram of crude product. In comparison, the weight of crude product from the reaction without benzene is 1.6 grams.

Analysis of the crude products by GC (SE-30 column) indicates that the amount of 1-pyrazinyl-1-propanone is nearly identical for each reaction. The reaction without benzene gives a significant amount of diacylation (ratio of mono- to diacylation - 6:1). In contrast, the ratio for the reaction run in the presence of benzene is 50:1.

The conversion demonstrates that the heterogeneous medium provides substantially monoacylation, whereas the homogeneous reaction conditions produce diacylation almost exclusively (as consistent with the results in Example XII).

CLAIMS

1. A process for preparing monoacylpyrazines characterised by providing a heterogeneous reaction medium consisting of a water-immiscible organic phase and an acidic aqueous phase, wherein the organic phase comprises a mixture of an aldehyde (R-CHO) and a pyrazine compound corresponding to the formula:

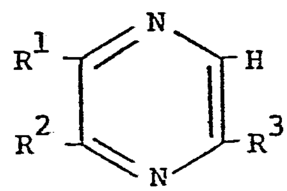

where $R^1$, $R^2$ and $R^3$ are substituents selected from hydrogen and alkyl groups, and $R^1$ and $R^2$ when taken together with connecting elements may form an alicyclic or aromatic structure, and R in the aldehyde compound is selected from aliphatic, alicyclic and aromatic groups; and maintaining efficient contact between the organic and aqueous phases for a period of time sufficient to achieve acylation of the pyrazine compound in the presence of a free radical generating agent and produce a monoacylpyrazine product corresponding to the formula:

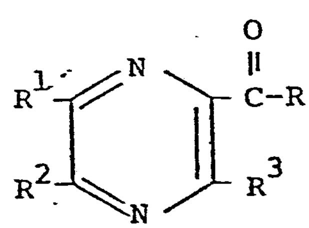

where R, $R^1$, $R^2$ and $R^3$ are as previously defined, which is then recovered.

2. A process in accordance with claim 1 characterised in that a water-immiscible solvent is present as a component of the organic phase.

3. A process in accordance with claim 1 or 2 characterised in that the aqueous phase has a pH in the range from 1 to 5.

4. A process in accordance with claim 1, 2 or 3 characterised in that the efficient contact between the organic and aqueous phases is accomplished by stirring.

5. A process in accordance with any of claims 1 to 4 characterised in that the heterogeneous reaction medium is maintained at a temperature between $0^o$ and $60^oC$ during the acylation reaction period.

6. A process in accordance with any of claims 1 to 5 characterised in that the free radical generating agent is a redox system.

7. A process in accordance with any of claims 1 to 6 characterised in that the aldehyde reactant is isovaleraldehyde, propionaldehyde, trimethylacetaldehyde, isobutyraldehyde, butyraldehyde, acetaldehyde or

p-methoxybenzaldehyde.

8. A process in accordance with any of claims 1 to 7 characterised in that the pyrazine reactant is pyrazine, 2,3-dimethylpyrazine, trimethylpyrazine, 2,6-dimethylpyrazine, 5,6,7,8-tetrahydroquinoxaline or isobutylpyrazine.

European Patent Office

# EUROPEAN SEARCH REPORT

0076085
Application number

EP 82 30 4973

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| | --- | | C 07 D 241/12 |
| D,A | JOURNAL OF THE CHEMICAL SOCIETY, Perkin II, 1972, London (GB) T. CARONNA et al.: "Homolytic acylation of protonated pyridine and pyrazine derivatives", pages 2035-2038 * page 2035, page 2036, table I; page 2038 * | 1,7,8 | C 07 D 241/42 |
| | ----- | | |
| | | | TECHNICAL FIELDS SEARCHED (Int. Cl. 3) |
| | | | C 07 D 241/12 |

The present search report has been drawn up for all claims

| Place of search | Date of completion of the search | Examiner |
|---|---|---|
| THE HAGUE | 11-01-1983 | FRANCOIS J.C.L. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82